# EUROPÄISCHE PATENTSCHRIFT

(11) **EP 0 297 415 B1**
(45) Veröffentlichungstag und Bekanntmachung des Hinweises auf die Patenterteilung: **04.08.1993**
(21) Anmeldenummer: 88109942.8
(22) Anmeldetag: 22.06.1988
(51) Int. Cl.: G01N 25/56, G01N 33/12

(54) **Verfahren und Vorrichtung zur Bestimmung des Wasseraktivitätswertes bei Lebensmitteln, insbesondere Fleischerzeugnissen**
Method and apparatus for determining water activity value in food, especially in meat
Procédé et appareil pour déterminer l'activité d'eau des aliments, en particulier de la viande

(30) Priorität: 30.06.1987 DE 3721511
(43) Veröffentlichungstag der Anmeldung: 04.01.1989
(73) Patentinhaber: Rödel, Wolfgang, Dr., D-95326 Kulmbach (DE)
(72) Erfinder: Rödel, Wolfgang, Dr., D-8650 Kulmbach (DE); Scheuer, Rainer, Dr., D-8650 Kulmbach (DE); Wagner, Hubertus, Dr., D-8581 Neudrossenfeld (DE)
(74) Vertreter: Flach, Dieter Rolf Paul, Dipl.-Phys.

(56) Entgegenhaltungen:
- EP-A- 0 144 443
- EP-A- 0 221 642
- DE-U- 8 217 261
- GB-A- 2 137 340

## Beschreibung

Die Erfindung betrifft ein Verfahren und eine Vorrichtung zur Messung des Wasseraktivitätswertes bei Fleischerzeugnissen nach dem Oberbegriff des Anspruches 1 bzw. 7.

Mit dem Verfahren und der Vorrichtung kann der Wasseraktivitätswert (aw-Wert) bei Fleischerzeugnissen gemessen werden. Der Wasseraktivitätswert ist ein Maß für die Verfügbarkeit des in einem Lebensmittel auf verschiedene Art gebundenen Wassers für Mikroorganismen. Die Wasseraktivität ist als die relative Feuchte definiert, die in der das Lebensmittel umgebenden Atmosphäre herrschen muß, damit sich hinsichtlich des Wasserdampfpartialdruckes in beiden Phasen ein Gleichgewicht einstellen kann. Der Wasseraktivitätswert wird in der Einheit 0 - 1.0 aw angegeben.

Die Wasseraktivität ist eine der Größen, die physikalische mechanische, chemische und mikrobiologische Eigenschaften eines Lebensmittels bestimmen. Der Wasseraktivitätswert stellt vor allem bei Fleischerzeugnissen einen wesentlichen Maßstab für die mikrobiologische Haltbarkeit dar. Die konservierende Wirkung zum Beispiel des Trocknens, Salzens und des Zuckerns beruht vor allem auf einer Erniedrigung des Wasseraktivitätswertes, wodurch für die unerwünschten Mikroorganismen, die Verderb oder Lebensmittelvergiftung verursachen können, die Lebensbedingungen erschwert bzw. völlig eingeschränkt werden. Für jede Mikroorganismenart gelten andere Grenz-aw-Werte. Diese individuellen Werte sind für die bei der Herstellung von Fleischerzeugnissen interessierenden Mikroorganismen hinreichend untersucht und bekannt. Durch die Kombination bestimmter aw-Werte mit anderen Konservierungsparametern im Fleischerzeugnis, wie Säuregrad (pH-Wert), Redoxpotential (Eh-Wert), Erhitzungswert (F-Wert), Starterkulturen usw., können Haltbarkeitszeiten abgeschätzt und entsprechende Fleischerzeugnisse produziert werden.

Aufgrund der Bedeutung des aw-Wertes vor allem für die mikrobiologische Haltbarkeit von Fleischerzeugnissen haben sich in den vergangenen Jahren die Methoden und Geräte zur Messung des aw-Wertes ständig verbessert. Für den aw-Bereich von Fleischerzeugnissen von ca. 0.99 bis 0.75 hat sich vor allem das Prinzip der elektrolytischen Meßzelle durchgesetzt, bei der die Widerstandsänderung eines Elektrolytgemisches in Abhängigkeit von der Feuchtigkeit der umgebenden Luft in einem geschlossenen Meßsystem zur Messung genutzt wird. Temperaturschwankungen während der Einstellung des Feuchtegleichgewichts (Dauer ca. 3 Stunden bei 25°C) zwischen Probe und Luft können das Ergebnis der Messung verfälschen, was aufwendige Temperieranordnungen notwendig macht. Darüber hinaus kommt es zur Meßwertverfälschung, wenn neben dem Wasserdampf andere dampfförmige Inhaltsstoffe des Lebensmittels auftreten (z. B. Glyzerin oder ähnliche Alkohole).

Ähnliche lange Meßdauern erfordern auch andere Meßverfahren wie beispielsweise sog. Taupunkt-Verfahren. Hier bestehen aber auch noch zusätzlich spezifische meßtechnische Probleme, vor allem wegen des für die Messung im allgemeinen notwendigen Luftstromes bei optoelektrischen Systemen. Andere außer Wasser im Lebensmittel vorkommende und verdampfende Inhaltsstoffe können auch bei diesem Verfahren den Meßwert stark verfälschen.

Schließlich sind auch kapazitive Messungen bekannt, bei denen über die in einem Kondensator eindringende Flüssigkeit der Probe das sich verändernde Dielektrikum bestimmt wird. Hier kann bei Erreichen der Sättigung eine Veränderung der Sensorcharakteristik auftreten, wodurch auch bei dieser Methode aufwendige Temperieranordnungen notwendig werden.

Aufgabe der vorliegenden Erfindung ist es von daher ausgehend vom Stand der Technik ein völlig neuartiges Meßverfahren sowie eine Vorrichtung zur Durchführung dieses Verfahrens zu schaffen und mit erhöhter Genauigkeit in vor allem erheblich kürzerer Zeit gegenüber dem Stand der Technik Wasseraktivitätswerte von bestimmten Lebensmitteln bzw. Gruppen von Lebensmitteln wie beispielsweise Fleischerzeugnisse zu bestimmen.

Die Aufgabe wird erfindungsgemäß bezüglich des Verfahrens entsprechend den im kennzeichnenden Teil des Anspruches 1 und bezüglich der Vorrichtung entsprechend den im kennzeichnenden Teil des Anspruches 7 angegebenen Merkmalen gelöst. Vorteilhafte Ausgestaltungen der Erfindung sind in den Unteransprüchen angegeben.

Durch die vorliegende Erfindung wird ein völlig neuartiger Weg beschritten, der in verblüffend kurzer Zeit beispielsweise nur von ca. 12 bis 15 Minuten eine exakte Bestimmung des Wasseraktivitätswertes (aw) mit einer 4-Stellen-Genauigkeit, d.h. bei einer Angabe in Prozent relativer Feuchtigkeit mit 2 Stellen hinter dem Komma - ermittelbar ist. Dies war bisher so nicht denkbar und muß vom Ergebnis her als durchaus überraschend bezeichnet werden.

Bei dem erfindungsgemäßen Verfahren wird der Wasseraktivitätswert (aw) über einen diesem Wert entsprechenden repräsentierenden Wert ermittelt. Als ein den Wasseraktivitätswert repräsentierender Wert gilt dabei der Gefrierpunkt einer zu untersuchenden Lebensmittelprobe. Durch Ermittlung des Gefrierpunktes also kann auf den Wasseraktivitätswert der Probe geschlossen werden. Dafür werden letztlich die Beziehungen
- zwischen dem Gefrierpunkt und der Kochsalzkonzentration der Lebensmittelprobe und
- zwischen der Kochsalzkonzentration und der Wasseraktivität herangezogen.

Die weitere Berechnung des eigentlichen aw-Wertes der Probe bei 25°C erfolgt über eine empirisch ermittelte mathematische Beziehung zwischen dem Wasseraktivitätswert am jeweiligen Gefrierpunkt und dem zugehörigen Wasseraktivitätswert bei einer Temperatur von 25°C. Diese empirisch ermittelbare mathematische Beziehung wird durch eine Eichung gewonnen, für deren Durchführung der Gefrierpunkt und der Wasseraktivitätswert mit herkömmlichen Verfahren an einer bestimmten Lebensmittelprobe bestimmt werden. Daraus lassen sich die Umrechnungsfaktoren und Umrechnungsgrößen ermitteln, mit denen bei weiter zu untersuchenden Lebensmittelproben aus dem ermittelten Gefrierpunkt dann der exakte Wasseraktivitätswert beispielsweise auch bei 25°C ermittelt werden kann. Ebenso kann aber der Schmelzpunkt als Extremwert zur Bestimmung des Wasseraktivitätswertes dienen. Dabei wird nach zuerst vorgenommener stärkerer Abkühlung über den Schmelzpunkt hinaus die zu untersuchende Probe langsam erwärmt, um darüber den Schmelzpunkt zu ermitteln.

Bei dem erfindungsgemäßen Verfahren wird ein zu untersuchendes Lebensmittelprodukt, beispielsweise ein Fleischerzeugnis stetig heruntergekühlt. Über einen eingesteckten Temperatursensor kann beispielsweise die Probentemperatur kontinuierlich registriert werden. Diese Werte werden einem Mikroprozessor zugeführt, der beispielsweise durch die Differenzierung der Zeit-Temperatur-Kurve während des Kühlprozesses den jeweiligen Gefrierpunkt der Probe exakt nach bestimmten Auswahlkriterien erkennen kann, um danach die Messung nach Umrechnung in den aw-Wert bei 25°C und Ausgabe des berechneten Wertes automatisch zu beenden. Das Verfahren eignet sich beispielsweise zur Bestimmung der Wasseraktivitätswerte von Fleischerzeugnissen in einem aw-Bereich von 0.999 bis 0.800.

Die erfindungsgemäße Vorrichtung ist so konzipiert, daß mit einfachen Mitteln und wenig Handgriffen die entsprechende Bestimmung des aw-Wertes erfolgen kann.

Das erfindungsgemäße Meßverfahren mit der erfindungsgemäßen Meßvorrichtung weist gegenüber herkömmlichen Meßverfahren, insbesondere dem elektrolytischen Meßverfahren bei 25°C folgende Besonderheiten auf:
- Für die Messung werden keine aufwendigen Temperierungsanlagen benötigt;
- Die Meßergebnisse sind auf mindestens ± 0,0002 aw reproduzierbar;
- die Meßdauer bei dem erfindungsgemäßen Verfahren und der zugehörigen Vorrichtung hängt von der jeweiligen Höhe des aw-Wertes ab und beträgt bei aw-Werten um 0.970 ca. 15 Minuten, bei aw-Werten um 0.800 ca. 20 Minuten;
- zudem kann die Meßdauer durch automatische Beendigung des Meßvorganges nach Ausgabe des ermittelten aw-Wertes optimiert werden; und
- vor allem weist das erfindungsgemäße Meßverfahren und die zugehörige Vorrichtung eine geringe Störanfälligkeit gegenüber probenspezifischen und externen Einflüssen auf.

Eine Vorrichtung zur Ermittlung des Fremdwassergehaltes in Milch ist grundsätzlich aus der DE-U 82 17 261 bekannt geworden. Dabei wird der Gefrierpunkt in einem Bereich zwischen 0^{o} bis 1^{o}C ermittelt, um eine möglicherweise unerlaubte Zumischung von Wasser zur Trinkmilch (Streckung) aufzudecken. Der Gefrierpunkt unverwässerter Milch ist bekannt (Basiswert -0.5350^{o}C). Bei Zugabe von Wasser zur Milch verschiebt, d. h. erhöht sich der Gefrierpunkt um wenige hundertstel Grad in Richtung 0^{o}C, wobei die Verschiebung dem umerlaubten Wasserzusatz, also dem Fremdwasserzusatz entspricht. Dabei bedeuten 10% Temperaturerhöhung gleich 10% Wasserzusatz.

Die Bestimmung des Fremdwasseranteils von Milch kann aber in keinster Weise mit der Methode der Bestimmung der relativen Feuchtigkeit noch mit der Bestimmung der sogeannten Wasseraktivität von sonstigen Lebensmitteln verglichen werden. Zudem ist auch keine Aussage über die mikrobiologische Stabilität möglich, da Milch generell mikrobiologisch instabil ist. Auch daraus ist ersichtlich, daß für einen Fachmann auf dem einschlägigen Gebiet die DE-U 82 17 261 nicht die geringsten Anregungen geben kann ein Verfahren sowie eine Vorrichtung zu entwickeln, um eine Aussage über die mikrobiologische Stabilität von Lebensmitteln zu erhalten.

Aber auch die aus der GB-A-2,137,340 vorbekannte Vorrichtung ist für einen völlig anderen Einsatzzweck gedacht und beschrieben. Bei dieser Vorveröffentlichung geht es um die Bestimmung und die Analyse der Zusammensetzung von Fleisch als solchem.

Weitere Vorteile, Einzelheiten und Merkmale der Erfindung ergeben sich nachfolgend aus dem anhand von Zeichnungen dargestellten Ausführungsbeispiel. Dabei zeigen im einzelnen
- Fig. 1 :: eine erfindungsgemäße Kühleinrichtung mit einem Probenbehälter in schematischer Vertikalschnittdarstellung;
- Fig. 2 :: ein Aufbauschema der elektronischen Meßeinrichtung zur Ermittlung des Wasseraktivitätswertes;
- Fig. 3a bis 3c :: drei typische Kurvenverläufe einer Temperatur-Zeit-Kurve bei Herabkühlen von zu untersuchenden Lebensmittelproben und
- Figur 4a bis 4c :: eine erfindungsgemäße Vorrichtung zum Einbringen von streichfähigen Lebensmittelproben in den Probenbehälter in Aufsicht- und schematischer Vertikalschnittdarstellung.

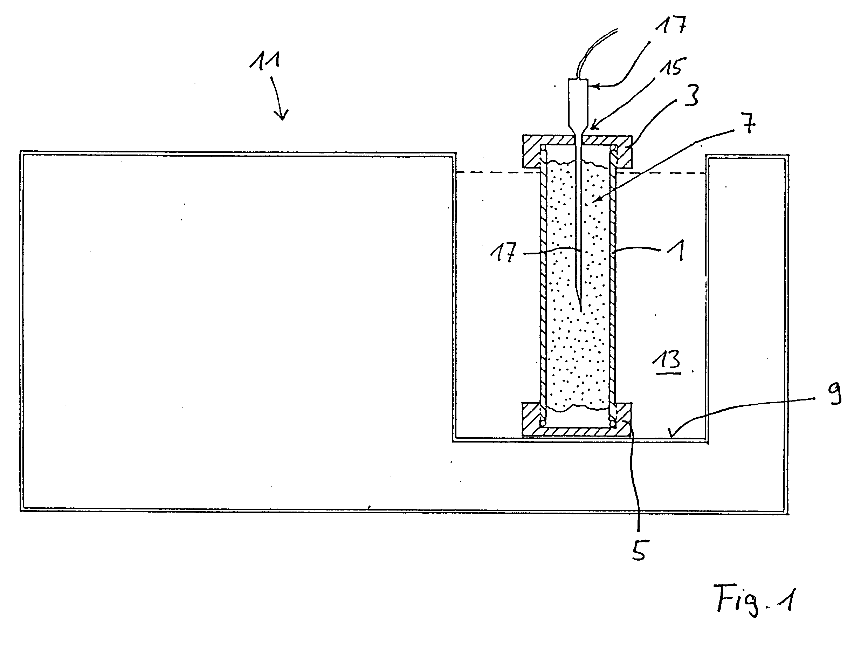

In Fig. 1 ist in Vertikaldarstellung ein beispielsweise röhrenförmiger Probenbehälter 1 gezeigt, der über einen oberen Deckel 3 verschließbar ist. Über den oberen Deckel 3 bzw. unteren Boden 5 kann in den Probenbehälter das zu untersuchende Lebensmittelerzeugnis, beispielsweise Fleischerzeugnis 7 zur Bestimmung des Wasseraktivitätswertes gegeben werden.

Der Probenbehälter 1 wird in eine wannenförmige Vertiefung 9 einer Kühlvorrichtung 11 gegeben, in welcher der Probenbehälter 1 zumindest annähernd über seine gesamte Höhe von einer Kühlflüssigkeit 13 umgeben ist. Die Kühlflüssigkeit 13 kann dabei beispielsweise aus technischem Alkohol bestehen.

Die Kühlvorrichtung 11 besteht insgesamt aus einem nicht näher in der Zeichnung dargestellten Kälteaggregat mit einem zugehörigen Kältebad, worüber letztlich die Kühlflüssigkeit und dann auch die Lebensmittelprobe 7 gekühlt wird. Das Kälteaggregat ist dabei so ausgelegt, daß eine Temperatur bis zu -40°C erzeugt werden kann.

Wie aus Fig. 1 ferner ersichtlich ist, kann über eine entsprechende Öffnung 15 im oberen Deckel 3 ein Temperatursensor 17 in die im Probenbehälter 1 befindliche und zu untersuchende Lebensmittelprobe 7 eingesteckt werden. Die Temperatur wird an der Fühlerspitze des Temperatursensors 17 gemessen.

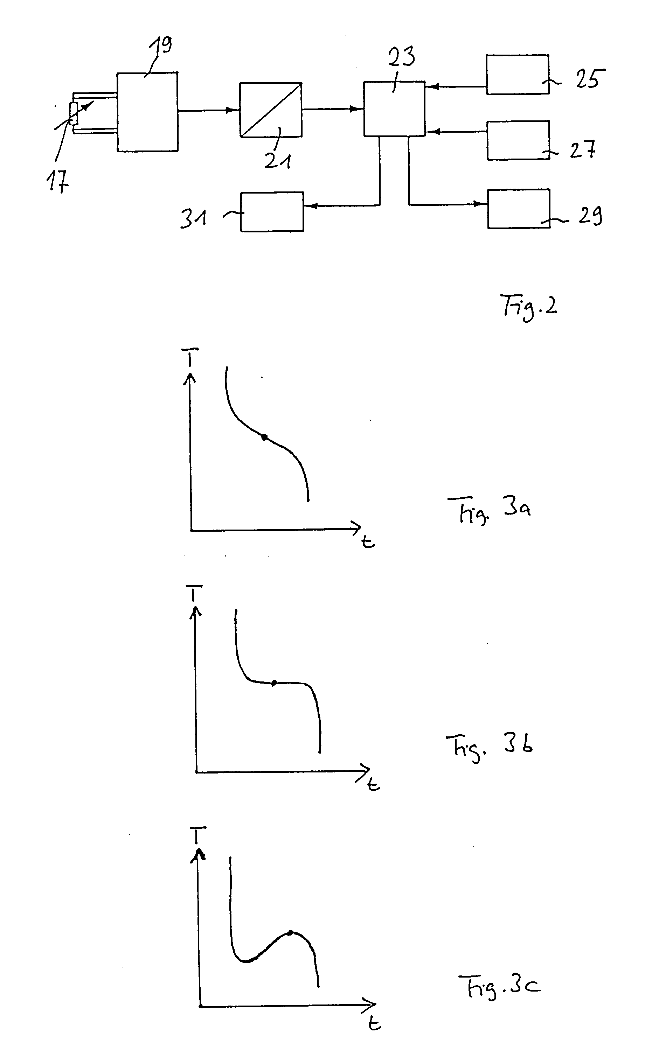

Fig. 2 gibt schematisch den Aufbau der elektronischen Meßeinrichtung wieder.

Die am Temperatursensor 17 gemessenen Werte werden an das zugehörige Meßgerät 19 weitergegeben. Die so ermittelten analogen Temperaturwerte werden über einen nachgeordneten Analog-/Digitalwandler 21 der eigentlichen, die Meßwerte verarbeitenden Elektronik zugeführt. Diese Elektronik besteht im gezeigten Ausführungsbeispiel aus einem Mikroprozessor 23 mit einem Eprom 25 zur Speicherung des Programms, einem Eingabetastenfeld 27 für Start/Stop, Daten, Probennummer und weiteren Kennzahlen, einer Digitalanzeige 29 für die ermittelten Meßwerte und weitere Informationen, sowie einem Ausgabefeld 31, beispielsweise in Form eines Bildschirms oder Protokolldruckers.

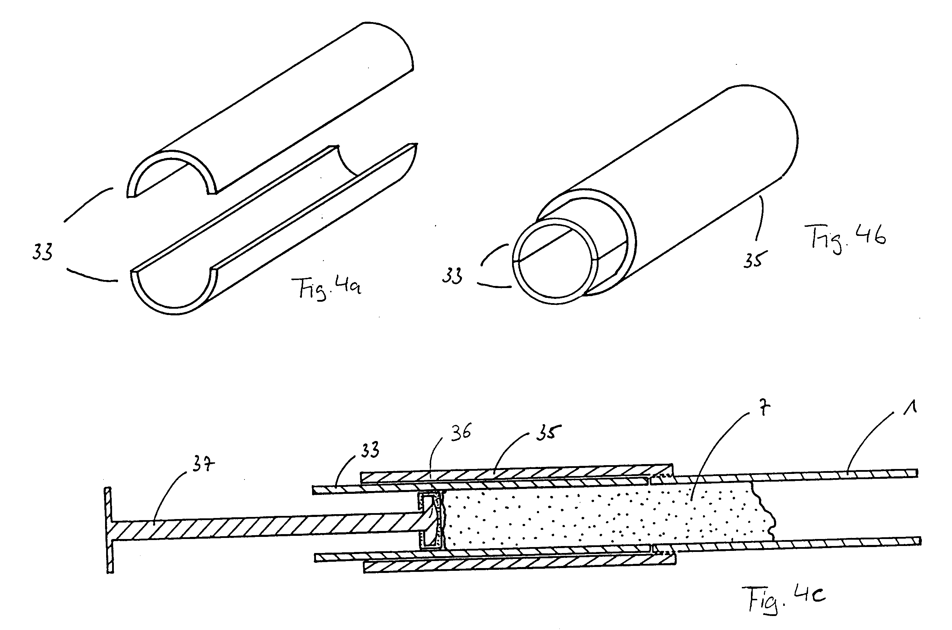

Anhand der Figuren 3a bis 3c wird nachfolgend der Ablauf der Bestimmung des Wasseraktivitätswertes erläutert.

In eine bis auf -35° bis -40°C gekühlte Kühlflüssigkeit 13 wird der beispielsweise oben und unten über die Schraubkappen 3 bzw. 5 verschlossene Probenbehälter in Form einer Kunststoffröhre bis etwa zur oberen Schraubkappe abgesenkt, nachdem die zu untersuchende Lebensmittelprobe 7 zunächst mit Hilfe der in Fig. 4a bis 4c gezeigten und nachfolgend noch erläuterten Vorrichtung in den Probenbehälter 1 eingebracht worden ist.
Danach wird der erwähnte Temperatursensor 17 in Form eines Nadelfühlers bis zum Zentrum der Lebensmittelprobe eingesteckt. Von dem Temperaturmeßgerät 19, mit dem der Temperatursensor 17 verbunden ist, werden zyklisch beispielsweise zweimal pro Sekunde Temperaturwerte ermittelt und an den Mikroprozessor 23 geleitet. Durch den Mikroprozessor 23 werden die so ermittelten Temperaturwerte, d.h. die Temperatur-Zeit-Kurve der gekühlten Lebensmittelprobe analysiert.

Durch entsprechend im Programm vorgewählte Kriterien kann der Gefrierpunkt der zu untersuchenden Lebensmittelprobe genauestens bestimmt und erkannt werden.

Dies kann erfindungsgemäß dadurch erfolgen, daß im Mikroprozessor 23 die Temperatur-Zeit-Kurve ständig differenziert und dadurch die Änderungen festgehalten werden. Wenn also die Steigung beispielsweise in Fig. 3a bzw. 3b von niedrigeren Werten bis auf einen Extremwert (bzw. Null) ansteigt und anschließend wieder kontinuierlich abnimmt, so ist bei der maximalen Steigung der Wendepunkt erreicht, der exakt dem Gefrierpunkt der zu untersuchenden Lebensmittelprobe 7 entspricht.

Wie in Figur 3c gezeigt ist, kann auch eine vorübergehende Unterkühlung eintreten. In diesem Fall entspricht der Gefrierpunkt dem Maximum der Gefrierkurve. Die Entscheidung, welches Auswahlkriterium zur Identifizierung des Gefrierpunktes zutrifft, wird bei diesem Verfahren, abhängig vom jeweiligen Kurvenverlauf, automatisch getroffen.

Die Messung wird beendet, wenn vor dem Durchfahren des Gefrierpunktes bestimmte Steigungswerte der Gefrierkurve überschritten und nach dem Durchfahren unterschritten werden (Figur 3a und 3b) bzw. wieder eine kontinuierliche Abnahme der Temperaturwerte registriert wird (Figur 3c).

Nach diesen bestimmten Auswahlkriterien kann der Gefrierpunkt genaustens erkannt und die Messung nach Umrechnung in den zu ermittelnden Wasseraktivitätswert bei 25°C und Ausgabe des berechneten Wertes automatisch beendet werden.

Sollte aufgrund eines selten vorkommenden atypischen Kurvenverlaufes der Gefrierpunkt nicht identifiziert und somit die Messung nicht automatisch beendet werden, so wird sie vom Programm abgebrochen, wenn die Probe eine Temperatur knapp oberhalb der Kühlbadtemperatur erreicht.

Nach Ausgabe des aw-Wertes ist die Meßvorrichtung wieder für eine neue Messung freigegeben.

Nachfolgend wird noch auf die Figuren 4a bis 4c näher eingegangen, in denen eine Vorrichtung zum Einbringen von streichfähigen Lebensmittelproben in den Probenbehälter in Aufsicht- und schematischer Vertikalschnittdarstellung gezeigt ist.

Zum Einbringen der streichfähigen Lebensmittelprobe in diesen Probenbehälter 1 werden zwei Halbröhren 33 und eine Rohrmanschette 35 mit kurzem Innengewinde an einer ihrer Stirnseitenöffnungen benötigt, worüber der Probenbehälter 1 über ein Außengewinde eingeschraubt wird. Ferner ist ein Kolben 36 mit einer Schiebestange 37 vorgesehen. Die beiden Halbröhren 33 werden mit der streichfähigen Lebensmittelprobe kantenhoch glatt gefüllt und danach die gefüllten Halbröhren so aufeinander gepaßt, daß eine vollständige Röhre mit gleichem Innendurchmesser wie der des Probenbehälters 1 entsteht. Über diese zusammengesetzte Röhre wird die Rohrmanschette 35 geschoben und in das kurze Innengewinde eines der beiden Außengewinde des beidseitig offenen Probenbehälters 1 eingeschraubt. Die pastenförmige Lebensmittelprobe 7 wird dann aus der Röhre mittels des Kolbens 36 und der Schiebestange 37 in den Probenbehälter 1 gedrückt.

Anschließend wird der Probenbehälter 1 beidseitig mit Deckeln 3 und 5 durch Verschrauben geschlossen. Hierdurch kann eine vollständige Befüllung des Probenbehälters 1 problemlos erzielt werden.

Möglich ist aber auch, daß ein in der Zeichnung nicht näher gezeigter zylinderförmiger Probenbefüller verwandt wird, der aus einem beidseitig offenen Metallrohr mit einem Innendurchmesser entsprechend der Länge des Probenbehälters besteht. Der Probenbefüller ist an seiner einen Stirnseitenöffnung scharf angeschliffen. Durch manuellen Druck kann er in eine entsprechend hohe Lebensmittelprobe eingedrückt werden, wodurch eine zylindrische Lebensmittelprobe ausgestanzt wird, die entweder in den Probenbehälter 1 manuell gedrückt oder mittels des in Figur 4c gezeigten Kolbens 36 mit der Schiebestange 37 eingeführt wird. In bestimmten Fällen kann auch der Probenbehälter 1 selbst zum Ausstechen der Lebensmittelprobe verwandt werden. Bei streichfähigen Lebensmittelproben eignet sich vor allem die Vorrichtung gemäß den Figuren 4a bis 4c. Bei schneidfähigen Lebensmittelproben ist demgegenüber die erwähnte Vorrichtung zu bevorzugen, mittels der eine Lebensmittelprobe ausgestanzt werden kann.

Ergänzend zu dem vorstehend erläuterten Ausführungsbeispiel wird angemerkt, daß als zu messender Extremwert, aus dem der Wasseraktivitätswert bestimmt werden kann, anstelle des Gefrierpunktes auch der Schmelzpunkt dienen kann. Dazu wird in der Regel bei gleichem apparativen Aufbau die zu untersuchende Lebensmittelprobe über den zu erwartenden Schmelzpunkt hinaus unterkühlt. Anschließend tritt ein langsamer Erwärmungsvorgang ein. Er kann durch Zuführung von Wärme durchgeführt werden.

Möglich ist aber allein die Verwendung von der Umgebungstemperatur. Dazu kann der gesamte Probenbehälter 1 wieder aus der Kühlflüssigkeit 13 herausgenommen werden, so daß dann die Umgebungstemperatur automatisch auf die zu untersuchende Lebensmittelprobe einwirkt und zu einer langsamen Temperaturerhöhung führt. Auch hier bildet sich wieder ein relativ langes stabiles Plateau aus, auf dem der als Extremwert zu messende Schmelzpunkt liegt.

## Patentansprüche

1. Verfahren zur Bestimmung des Wasseraktivitätswertes von Lebensmitteln, insbesondere Fleischerzeugnissen, **dadurch gekennzeichnet**, daß bei einer zu untersuchenden Lebensmittelprobe als ein den Wasseraktivitätswert repräsentierender Wert der lebensmittelprobenabhängige Gefrierpunkt und/oder Schmelzpunkt gemessen oder ermittelt wird, aus welchem mittels einer durch Eichung herleitbaren empirischen Umrechnungen der Wasseraktivitätswert ermittelbar ist.

2. Verfahren nach Anspruch 1, **dadurch gekennzeichnet,** daß die zu untersuchende Lebensmittelprobe abgekühlt wird, daß während des Abkühlvorganges die Temperatur in der zu untersuchenden Lebensmittelprobe kontinuierlich bzw. in diskontinuierlichen Zeitabständen gemessen wird, wobei die Temperaturmessung bis über den, den Gefrierpunkt darstellenden Extremwert der gemessenen Temperatur-Zeit-Kurve hinaus erfolgt und nach Erreichen dieses Extremwertes beendet oder knapp oberhalb der Kühlbadtemperatur abgebrochen wird.

3. Verfahren nach Anspruch 1, **dadurch gekennzeichnet,** daß die zu untersuchende Lebensmittelprobe bis unter den Schmelzpunkt abgekühlt wird, daß anschließend die soweit abgekühlte Lebensmittelprobe vorzugsweise durch Einwirkung der Umgebungstemperatur langsam erwärmt, wobei während des nachfolgenden Erwärmungsvorganges die Temperatur in der zu untersuchenden Lebensmittelprobe kontinuierlich bzw. in diskontinuierlichen Zeitabständen gemessen wird und die Temperaturmessung bis über den, den Schmelzpunkt darstellenden Extremwert der gemessenen Temperatur-Zeit-Kurve hinaus erfolgt und nach Erreichen und Überschreiten dieses Extremwertes beendet wird.

4. Verfahren nach Anspruch 1, 2 oder 3, **dadurch gekennzeichnet,** daß die zu untersuchende Lebensmittelprobe in ein Kältebad gegeben wird, dessen Temperatur weit unterhalb des zu messenden Gefrierpunktes, vorzugsweise unter -20°C bzw. -30°C liegt.

5. Verfahren nach einem der Ansprüche 1 bis 4, **dadurch gekennzeichnet,** daß die elektrisch/elektronisch gemessene Temperatur-Zeit-Kurve zur Bestimmung des den Gefrier- oder Schmelzpunkt darstellenden Extremwertes in einer Differenzierstufe (Mikroprozessor) differenziert wird.

6. Verfahren nach einem der Ansprüche 1 bis 5, **dadurch gekennzeichnet,** daß in einer Vergleichsschaltung bzw. -steuerung der Verlauf der Temperatur-Zeit-Kurve vor und nach Erreichen des Gefrier- oder Schmelzpunktes/Extrempunktes registriert wird und nach Über- bzw. Unterschreiten eines empirisch ermittelten Grenzwertes für die Steigung bzw. nach kontinuierlicher Abnahme der Temperatur die Messung selbständig beendet wird.

7. Vorrichtung zur Durchführung des Verfahrens nach einem der Ansprüche 1 bis 6, unter Verwendung einer Kühlvorrichtung (11) für die zu untersuchende Lebensmittelprobe (7), mit einem Temperatursensor (17) zur Messung der in der Lebensmittelprobe (7) herabkühlbaren Temperatur, wobei mit der Kühlvorrichtung (11) Temperaturen bevorzugt weit unterhalb des Gefrierpunktes der zu untersuchenden Lebensmittelprobe (7), d.h. bevorzugt unterhalb -20^{o}C erreichbar sind, und wobei dem Temperatursensor (17) bzw. dessen zugehörigem Meßgerät (19) eine Differenzierschaltung zur Bestimmung des als Extremwert in der über den Temperatursensor (17) gemessenen Temperatur-Zeit-Kurve identifizierten Gefrier- oder Schmelzpunktes nachgeschaltet ist.

8. Vorrichtung nach Anspruch 7, **dadurch gekennzeichnet**, daß die Differenzierschaltung aus einem Mikroprozessor (23) besteht.

9. Vorrichtung nach Anspruch 7 oder 8, **dadurch gekennzeichnet,** daß zwischen dem Temperatursensor (17) bzw. dessen Meßgerät (19) und der Differenzierstufe, vorzugsweise in Form des Mikroprozessors (23), ein Analog-Digital-Wandler (21) zwischengeschaltet ist.

10. Vorrichtung nach einem der Ansprüche 7 bis 9, **dadurch gekennzeichnet**, daß die Kühlvorrichtung (11) eine vorgekühlte, die Lebensmittelprobe aufnehmende Kühlflüssigkeit (13) aufweist.

11. Vorrichtung nach einem der Ansprüche 7 bis 10, **dadurch gekennzeichnet,** daß die Kühlflüssigkeit (13) der Kühlvorrichtung (11) Temperaturen von weniger als -20°C, vorzugsweise weniger als -30°C aufweist.

12. Vorrichtung nach einem der Ansprüche 7 bis 11, **dadurch gekennzeichnet,** daß die wannenförmige Vertiefung (9) zur Aufnahme der in eine Kühlflüssigkeit (13) einbringbaren, zu untersuchenden Lebensmittelprobe (7) von einem weiteren, durch ein Kälteaggregat vorkühlbaren Kältebad umgeben ist.

13. Vorrichtung nach einem der Ansprüche 7 bis 12, **dadurch gekennzeichnet,** daß der Temperatursensor in einen Probenbehälter (1) und damit in die in den Probenbehälter (1) einbringbare Lebensmittelprobe (7) einsteckbar ist.

14. Vorrichtung, nach einem der Ansprüche 7 bis 13, zum Einbringen von streichfähigen Lebensmittelproben in einen Probenbehälter, **gekennzeichnet durch** zwei mit einer streichfähigen Lebensmittelprobe kantenhoch glatt befüllbare Halbschalen (Halbröhren 33), deren Innenquerschnitt dem Innenquerschnitt des Probenbehälters (1) entspricht, undeinen Kolben (36) mit Schiebestange (37) zum Eindrücken der streichfähigen Lebensmittelprobe aus den beiden zusammengefügten Halbschalen (33) in den Probenbehälter (1)

15. Vorrichtung nach Anspruch 14, **dadurch gekennzeichnet,** daß zur Fixierung der beiden Halbschalen (Halbröhren 33) eine die beiden Halbschalen aufnehmende und an dem Probenbehälter (1) sich abstützende bzw. außen eindrehbare Fixiermanschette (35) vorgesehen ist, deren Innenquerschnitt dem Außenquerschnitt der zusammengefügten Halbschalen (33) entspricht.

16. Vorrichtung, nach einem der Ansprüche 7 bis 13, zum Einbringen von schneidfähigen Lebensmittelproben in einen Probenbehälter, **gekennzeichnet durch** einen stirnseitig angeschliffenen Probenbehälter (1) bzw. stirnseitig angeschliffenen, ein Ausstanzen der zu untersuchenden Lebensmittelprobe (7) ermöglichenden und dem Innenquerschnitt des Probenbehälters (1) zum Umfüllen der ausgestanzten Lebensmittelprobe (7) in den Probenbehälter (1) entsprechenden Probenbefüller.

## Claims

1. A method for determining the water activity value of foodstuffs and more particularly of meat products, characterized in that in the case of a foodstuff sample to be investigated the foodstuff sample-dependent freezing point and/or fusing point is measured or ascertained as being representation of the water activity value, from which by means of an empirical calculation able to be derived by calibration the water activity value is able to be ascertained.

2. The method as claimed in claim 1, characterized in that the foodstuff sample to be investigated is cooled, that during the process of cooling the temperature in the foodstuff sample to be investigated is measured continuously or, respectively, in discontinuous time intervals, the measurement of the temperature being performed as far as or, respectively, past the extreme value representing the freezing point, of the measured temperature time curve and after reaching such extreme value the temperature measurement is terminated or is interrupted just above the temperature of the cooling bath.

3. The method as claimed in claim 1, characterized in that the foodstuff sample to be investigated is cooled as far as a temperature under the fusing point, in that after this the foodstuff sample cooled down so far is slowly warmed, preferably by the action of the ambient temperature, and during the following warming up operation the temperature in the foodstuff sample to be investigated is measured continuously or, respectively, in discontinuous time intervals, and the measurement of temperature is performed as far as or, respectively, past the extreme value representing the freezing point, of the measured temperature time curve and after reaching such extreme value the temperature measurement is terminated.

4. The method as claimed in claim 1, claim 2 or claim 3, characterized in that the foodstuff sample to be investigated is placed in a refrigerating bath whose temperature is far below the freezing point to be measured and preferably under -20° C or, respectively, under -30° C.

5. The method as claimed in any one of the claims 1 through 4, characterized in that the electrically/electronically temperature time curve for the determination of the extreme value representing the freezing or fusing point is differentiated in a differentiating means (i. e. a microprocessor).

6. The method as claimed in any one of the claims 1 through 5, characterized in that in a comparison circuit or, respectively, a controlling means, the temperature time curve is registered prior to and after reaching the freezing or fusing point/extreme point and after exceeding or, respectively, going below an empirically ascertained limit value for the inclination or, respectively, after a continuous reduction in temperature the measurement is automatically halted.

7. An apparatus for the performing the method as claimed in any one of the claims 1 through 6, using a cooling device (11) for the foodstuff sample (7) to be investigated, comprising a temperature sensor (17) for the measurement of the temperature obtaining in the foodstuff sample (7) able to be cooled,, the cooling device (11) making it possible to reach temperatures which are preferably far below the freezing point of the foodstuff sample (7) to be investigated, that is to say preferably below -20° C, the temperature sensor (17) or, respectively, the measuring instrument (19) thereof being followed by a differentiating circuit for the ascertainment of the freezing point or fusing point able to be identified as an extreme value in the temperature time curve measured using the temperature sensor (17).

8. The apparatus as claimed in claim 7, characterized in that the differentiating circuit consists of a microprocessor (23).

9. The apparatus as claimed in claim 7 or claim 8, characterized in that between the temperature sensor (17) or, respectively, its measuring instrument (19) and the differentiating means, preferably in the form of the microprocessor (23) an analog/digital converter (21) is arranged.

10. The apparatus as claimed in any one of the claims 7 through 9, characterized in that the cooling device (11) comprises a pre-cooled cooling liquid (13) receiving the foodstuff sample.

11. The apparatus as claimed in any one of the claims 7 through 10, characterized in that the cooling liquid (13) of the cooling device (11) has a temperature of less than -20° C and preferably -30° C.

12. The apparatus as claimed in any one of the claims 7 through 11, characterized in that the trough-like depression (9) is surrounded by a further refrigerating bath, which is pre-cooled by a refrigerating unit, in order to receive the foodstuff sample to be investigated and able to be placed in a cooling liquid (13).

13. The apparatus as claimed in any one of the claims 7 through 12, characterized in that the temperature sensor is able to be inserted into sample container (1) and consequently into the foodstuff sample (7) able to be placed in the sample container (1).

14. The apparatus as claimed in any one of the claims 7 through 13, adapted for the introduction of spreadable foodstuff samples into a sample container, characterized by two half shells (half tubes 33) adapted to be filled with a spreadable foodstuff sample smoothed off at the top edge thereof, the inner cross section of the half shells corresponding to the inner cross section of the sample container (1), and a piston (36) with a thrust rod (37) for pressing the spreadable foodstuff sample out of the two assembled half shells (33) into the sample container (1).

15. The apparatus as claimed in claim 14, characterized in that for fixing the two half shells (half tubes 33) a fixing sleeve (35) is provided receiving the two half shells and bearing against the sample container (1) or, respectively, externally able to be screwed onto it, the cross section thereof corresponding to the external cross section of the half shells (33) when joined together.

16. The apparatus as claimed in any one of the claims 7 through 12 for the introduction of foodstuff samples, which are able to be cut, into a sample container, characterized by a sample container (1), which is sharpened at its end, or, respectively, a sample charging device, which is sharpened at its end, and makes possible a stamping out of the foodstuff sample (7) to be investigated and corresponding to the inner cross section of the sample container (1) for transferring the stamped out foodstuff sample (7) into the sample container (1).

## Revendications

1. Procédé pour déterminer l'activité d'eau des aliments et en particulier des produits à base de viande, caractérisé à ce que le point de congélation et/ou le point de fusion propre à un échantillon de denrée alimentaire et constituant une valeur représentative de l'activité d'eau de celle-ci est mesuré ou déterminé, tandis que la valeur de l'activité d'eau peut être déterminée par des conversions empiriques basées sur un étalonnage.

2. Procédé selon la revendication 1, caractérisé en ce que l'échantillon de denrée alimentaire à examiner est refroidi, que pendant le processus de refroidissement, la température de l'échantillon de denrée alimentaire à examiner est mesurée en continu ou à des intervalles de temps discontinus, tandis que la mesure de température a lieu jusqu'au-delà de la valeur extrême représentant le point de congélation de la courbe mesurée température-temps et que, quand cette valeur extrême est atteinte, la mesure est interrompue ou est arrêtée un peu au-dessus de la température du bain de refroidissement.

3. Procédé selon la revendication 1, caractérisé en ce que l'échantillon de denrée alimentaire à examiner est refroidi jusqu'en-dessous du point de fusion, que l'échantillon de denrée alimentaire ainsi refroidi est réchauffé lentement sous l'effet de la température ambiante tandis que, pendant le processus d'échauffement ultérieur, la température dans l'échantillon de denrée alimentaire à examiner est mesurée en continu ou à des intervalles de temps discontinus et que la température de mesure a lieu jusqu'au delà de la valeur extrême de la courbe température-temps mesurée, qui représente le point de fusion et que cette mesure est interrompue quand cette valeur extrême a été atteinte et dépassée.

4. Procédé selon la revendication 1, 2 ou 3 caractérisé en ce que l'échantillon de denrée alimentaire à examiner est placé dans un bain de refroidissement dont la température se situe loin en-dessous du point de congélation à mesurer et de préférence en-dessous de -20°C ou de -30°C.

5. Procédé selon l'une des revendications 1 à 4, caractérisé en ce que la courbe température-temps mesurée par une méthode électrique ou électronique est différentiée pour déterminer la valeur extrême représentant le point de congélation ou le point de fusion par une opération de différentiation (micro-processeur).

6. Procédé selon l'une des revendications 1 à 5, caractérisé en ce que, au cours d'une opération de comparaison ou de réglage, l'allure de la courbe température-temps est enregistrée avant et après que le point extrême de fusion ou de congélation ait été atteint et après dépassement par le dessus ou par le dessous d'une valeur limite déterminée pour la pente ou pour la diminution continue de la température, la mesure est interrompue automatiquement.

7. Dispositif pour mettre en oeuvre le procédé selon l'une des revendications 1 à 6 en utilisant un dispositif de refroidissement 11 pour l'échantillon de denrée alimentaire examiné 7, avec un détecteur de température 17 pour mesurer la température de refroidissement dans l'échantillon de denrée alimentaire 7, tandis que le dispositif de refroidissement 11 permet d'atteindre des températures qui sont, de préférence loin au dessous du point de congélation de l'échantillon de denrée alimentaire examiné 7, c'est-à-dire de préférence au-dessous de -20°C et tandis que le détecteur de température 17 ou son appareil de mesure correspondant 19 est suivi d'un circuit de différentiation pour déterminer le point de congélation ou de fusion identifié comme étant la valeur extrême de la courbe température/temps mesurée au moyen du détecteur de température.

8. Appareil selon la revendication 7 caractérisé en ce que le circuit de différentiation est constitué d'un micro-processeur 23.

9. Appareil selon la revendication 7 et 8, caractérisé en ce que un convertisseur analogique 21 est interposé entre le détecteur de température 19 ou son appareil de mesure 19 et l'étage de différentiation, par exemple sous forme du micro-processeur 23.

10. Appareil selon l'une des revendications 7 à 9, caractérisé en ce que le dispositif de refroidissement 11 comporte un liquide de refroidissement 13 pré-refroidi qui reçoit l'échantillon de denrée alimentaire.

11. Dispositif selon l'une des revendications 7 à 10, caractérisé en ce que le liquide de refroidissement 13 du dispositif de refroidissement 11 présente des températures inférieures à -20°C et de préférence inférieures à -30°C.

12. Appareil selon l'une des revendications 7 à 11, caractérisé en ce que le renfoncement 9 en forme de cuvette, destiné à recevoir l'échantillon de denrée alimentaire à examiner 7 pouvant être introduit dans le liquide de refroidissement 13, est entouré par un autre bain de refroidissement pouvant être refroidi d'avance par un groupe frigorifique.

13. Appareil selon l'une des revendications 7 à 12, caractérisé à ce que le détecteur de température peut être inséré dans un récipient à échantillon 1 et aussi dans l'échantillon de denrée alimentaire 7 pouvant être placé dans le récipient échantillon 1.

14. Dispositif selon l'une des revendications 7 à 13, pour introduire des échantillons de denrées alimentaires tartinables dans un récipient à échantillon, caractérisé par deux demi-coquilles pouvant être remplies à ras bord de l'échantillon de denrée alimentaire tartinable (demi-tube 33) dont la section intérieure correspond à la section intérieure du récipient à échantillon et un piston 36 avec une tringle de poussée 37 pour pousser l'échantillon de denrée alimentaire tartinable hors des deux demi-coquilles assemblées 33 dans le récipient à échantillon 1.

15. Appareil selon la revendication 14 caractérisé en ce que, pour fixer les deux demi-coquilles (demi-tubes 33) il est prévu une manchette de fixation 35 recevant les deux demi-coquilles et pouvant s'appuyer ou s'emboîter extérieurement sur le récipient à échantillon 1, cette manchette ayant une section intérieure qui correspond à la section extérieure des demi-coquilles assemblées 33.

16. Appareil selon l'une des revendications 7 à 13, pour introduire les échantillons de denrées alimentaires découpables dans un récipient à échantillon, caractérisé par un récipient à échantillon 1 aiguisé frontalement, par un chargeur d'échantillon aiguisé frontalement permettant d'extraire par estampage l'échantillon de denrée alimentaire examiné 7 et correspondant à la section intérieure du récipient à échantillon 1 pour transférer l'échantillon de denrée alimentaire extrait par estampage 7 dans le récipient à échantillon 1.
